# EUROPEAN PATENT SPECIFICATION

(11) **EP 0 875 758 B1**
(45) Date of publication and mention of the grant of the patent: **01.08.2001**
(21) Application number: 98108095.5
(22) Date of filing: 04.05.1998
(51) Int. Cl.: G01N 33/543, G01N 33/68

(54) **Immunoassay**
Immunoassay
Immunoessai

(30) Priority: 02.05.1997 GB 9708961
(43) Date of publication of application: 04.11.1998
(73) Proprietor: Ringel, Karl-Peter, Prof. Dr., Dublin 14 (IE)
(72) Inventor: Ringel, Karl-Peter, Prof. Dr., Dublin 14 (IE)
(74) Representative: Wain, Christopher Paul

(56) References cited:
- EP-A- 0 504 432
- WO-A-85/02262
- DE-A- 4 123 324
- DE-A- 4 329 791
- FR-A- 2 377 629

## Description

This invention relates to a method and apparatus for carrying out an immunoassay, particularly but not exclusively for in vitro testing of allergy.

Many immunoassays require a separation step. In order to facilitate this, it is known to insolubilise a reagent by binding it to a water-insoluble support so that any reaction product formed between the reagent and the sample during the immunoassay is held on the support. Then, either the support or the remaining reaction solution can be independently tested separately from the other, to establish, directly or indirectly, the presence and/or amount of the analyte under assay. Examples of such systems include the use of allergen-coated paper discs in the well known RAST (trademark) radioimmunoassays for in vitro testing of allergy.

In the RAST (trademark) allergy test, there are used paper discs to which have been bound a single allergen. It is therefore necessary, when conducting a batch of tests for, say, 50 different allergens on a single sample, to select the appropriate 50 different paper discs, to place each in a reaction analysis vessel, and then to proceed with the test in each of the 50 different vessels. The storing, retrieving, handling and placing up to 600 or so differently coated discs (there being this number of allergens for which assays are needed) in individual vessels as required for the selected analyses, is complex. The discs are not easily handled and it is difficult to provide reliable automation for this complex part of the overall sample analysis.

We have how devised a simpler way of carrying out this type of assay whereby the above-mentioned particular complexities associated with the use of allergen coated paper discs are avoided or overcome.

In accordance with a feature of the present invention, plain paper discs are placed in the individual reaction analysis vessels where they are then coated with the appropriate antigen immediately before the assay is conducted in the vessel. In this way, the necessity to make and store 600 or so differently coated paper discs is avoided. Instead, it is necessary to store and be able selectively to dispense 600 or so different allergen solutions into the individual reaction analysis vessels containing the blank discs. However, the handling of liquid reagents and their dispensation into reaction vessels are well established techniques easily capable of automation. Accordingly, the invention enables a simplification of the overall procedure which may, if desired, be automated.

Whilst the invention is particularly useful in allergy testing and will be described hereafter in that connection, it is to be understood that it can also be used for other banks of tests.

According to one aspect of the invention, there is provided a method of immunoassay of an analyte in a liquid sample, which comprises the steps of:
a) providing a plate in a vessel which plate will react when brought into contact with liquid first reagent in step (b);
b) adding to the vessel liquid first reagent which reacts with the surface of the plate to become bound thereto;
c) adding the liquid sample containing the analyte under assay to the vessel, whereby the analyte is immunospecifically bound directly or indirectly with said first reagent on said plate; and
d) directly or indirectly assaying the analyte bound to the plate.

According to another aspect, the invention provides apparatus for conducting a plurality of identical or similar tests on a biological fluid, for a plurality of analytes therein, wherein each test sample is contacted with a specific reagent for that test bound to an insoluble support, which apparatus comprises a plurality of reaction vessels each containing an identical reaction plate; means for selectively dispensing into each vessel one of a plurality of first liquid reagents; means for aspirating liquid from each vessel; means for dispensing liquid reagent into each vessel; and means for assaying a component in each vessel.

In step (a) of the method of the invention, a reactive plate is provided in a vessel. The plate is not fixed but is loose in the vessel. The plate will usually be flexible. It is preferably of paper, preferably highly porous paper but, instead of being of paper, the plate may be of a flexible or rigid plastics material, such as a plastics material made by a condensation or addition reaction, for example polyamide or polystyrene. Preferably, the plate will lie in the bottom of the vessel and, since most vessels are of circular section, the plate can advantageously be disc-like of a diameter slightly less than the inner diameter of the vessel so as to be fairly snugly received in the vessel. The exact shape of the plate is not critical but a planar disc is preferred.

The plate must be reactive in the sense that it will react when brought into contact with liquid first reagent in step (b). The activation of paper and plastics substrates so as to render them autoreactive towards biological reagents such as antigens and antibodies and other binding proteins is well known in the art. In the case of paper or like substrates, the method involves activating the substrate surface by reacting it with, for example, cyanogen bromide, or an epoxide, or by diazotisation, to provide reactive sites thereon for subsequent covalent bonding to the first reagent. The bonding of, for example, allergens to paper discs is well known in the art and the activation of the paper surface used in the present invention can be the same as that used in the known prior procedures. The allergens are mostly proteins, glycoproteins, lipoproteins and some haptens bound to human serum albumin (HSA). The amino, carbonyl sulphur and other groups therein bind covalently to the activated surface of the plate.

By way of example, the paper discs can be activated by the NIBSC (National Institute for Biological Standards and Controls, U.K) method as follows. The discs are first allowed to swell in water in a vessel for about 30 minutes. After this, a 5% solution of cyanogen bromide is added, and mixed in the vessel at room temperature for about 10 minutes using a mechanical stirrer. Sodium hydroxide (0.05M) is added to maintain the pH in the range 10.25 to 10.50. The mixture is then poured into cold sodium bicarbonate solution and the discs separated and washed with sodium carbonate solution (5x) and then with acetone (4x). The discs are then dried overnight under nitrogen and are then ready for use in the assay of the invention. Alternative procedures are also known which for example use a different buffer, eg. potassium phosphate, and a different pH range, eg. 11.0 to 12.0.

Activated plastics materials such as polystyrene, polyamide or nylon, are commercially available, eg. from Nunc GmbH, Wiesbaden, Germany, Pall Bio Support Div., Port Washington, New York, U.S.A., or Gelman Sciences Ltd., Northampton, U.K.

It is highly preferred in accordance with the invention, to make the activated plates in advance of their use in the assay. Thus, they can be made in bulk and stored relatively easily, for example in reasonably air-tight containers at about 4°C for up to about two years. Over this period, they will maintain their auto-reactivity. In accordance with a highly preferred feature of the invention, the activated plates are placed individually in analysis vessels, eg. microtitre plate wells, and the wells closed so that the activated plates are stored therein ready for the assay. It is notable that, by contrast, the allergen-coated discs used in the prior art must either be stored in freeze-dried condition (in which case they must be reconstituted prior to use) or they must be kept, at all times prior to use, in a buffer.

Whilst the use of pre-activated plates is highly preferred, it is possible to carry out the activation of a plate in the reaction vessel before step (a) of the immunoassay. This is not preferred but it is possible.

In German patent no. 4123324, there is described a disc having antigens or antibodies attached thereto, which disc is especially useful in allergy tests. The unique feature of the disc is that it has a substantially central hole or aperture, and is thus ring-shaped. The advantage of this disc is that, when used in a colorimetric or fluorescence assay, the final spectral test can be made directly on the liquid in the test vessel by making the observation on a light path through the hole or aperture in the disc. This obviates the need (essential with non-apertured discs) to remove liquid or the disc from the vessel before making the spectral test.

Such discs are highly preferred for use in the present invention and reference should be made to DE 4123324 C for full details. In accordance with the present invention, these discs (hereinafter particularly referred to as "rings" for simplicity but without limitation) have the respective allergen (or other reagent) bound thereto in step (b) of the method. In step (a), an activated disc is provided in the test vessel ready for binding to the allergen. The rings not only provide the advantage referred to above, but also provide further and unexpected advantages in step (b) of the method, to be described hereafter.

As will be well known to those skilled in the art, allergy tests are normally effected using banks or trays of reaction vessels, eg. in microtitre plates containing for example 96 individual reaction vessels. The method of the invention can, of course, be carried out in this way, with different allergens being supplied to each vessel, for each sample to be tested, in step (b) and with aliquots of the same sample being supplied to each such vessel in step (c). The use of microtitre plates and the like for simultaneously effecting a plurality of assays is, of course, well known and will not be further described herein.

In step (b) of the process of the invention, liquid first reagent is added to the reaction vessel (containing the reactive plate) whereby the first reagent reacts with the surface of the plate to become bound thereto. As will be understood, in the case of allergy testing, the said first reagent will be an allergen (i.e. the allergen in respect of which allergenicity is to be tested). The allergen becomes bound to the plate surface. In the case of assays other than allergy assays, the first reagent will be selected appropriately as will be clear to those skilled in the art.

In the prior known preparation of allergen coated paper discs for use in the RAST (trademark) process, for example, reaction between an allergen and the paper disc surface usually takes several hours (eg. up to 24 hours) to be complete. This long period of time is not particularly disadvantageous in the prior art procedures since the coated discs are made in advance of the assay and are then stored. However, in the process of the present invention, such a period would be a disadvantage since the coating of the discs is part of the assay process itself. We have found that this problem can be overcome by using accelerators and/or catalysts for the binding reaction. In this way, the binding can be completed much more quickly, for example within about 60 minutes. Suitable accelerators and catalysts will be known to those skilled in the art, but they include, for example, peroxides and various other free radical generators or catalysts.

The accelerators and/or catalysts can advantageously be included in the liquid first reagents, and thus dispensed therewith into the reaction vessels to contact therein the activated plate surface. Thus, the use of accelerators and/or catalysts in no way complicates the process because they can be admixed with the liquid first reagent.

By way of example, activated rings can be coated with allergen as follows. The rings are placed (for storage) in the individual wells of a microtitre plate. Into each well is delivered 50 *µ*l liquid allergen solution containing an accelerator. The wells are then subjected to orbital shaking for 50 to 60 minutes at room temperature. After this, about 50*µ*l of a blocking solution, e.g. epichlorhydrin or diethanolamine solution, were delivered to each well, and the wells shaken for a further 20 to 30 minutes at room temperature. Finally, the coated rings were washed (3x) with wash solution.

The liquid allergens (extracts) are preferably prepared and tested according to the "Method of the Laboratory of Allergenic Products DBP,OBRR, CDB and FDA 1986", and Standard Methods used at NIBSC for control of allergen extracts. All the extracts used for coupling to a solid phase are preferably tested by an appropriate procedure to ensure suitability. For pollen extracts, an isoelectric focussing pattern similar to that of the OBRR Reference preparation is the minimal requirement. For Dermatophagoides extracts, demonstration of similarity to the OBRR Reference preparation by RAST (trademark) inhibition is a minimum requirement. For food extracts, demonstration of similarity to the commercially purchased extracts (Prick test solution) by RAST (trademark) inhibition is a minimum requirement. An example of the allergen extraction procedure is as follows:
1. 4 grams of source material are extracted with 40 ml of suitable extracting fluid at 4°C with gentle stirring or rotation for 20 hours.
2. The mixture is centrifuged at 10,000 x g at 4°C for 30 minutes.
3. The supernatant is filtered through a 0.22 membrane filter.
4. The filtrate is dialysed against buffer in a dialysis tubing MWCO 7,000.
5. Stabilizer and accelerators/catalysts are added.

After reaction between the first reagent and the activated plate surface, the liquid reagent in the vessel is removed, usually by aspiration, and the vessel and plate are then washed. In order to ensure that there are no active sites remaining on the plate surface (which sites could interfere with subsequent assay steps), the plate can advantageously be washed with a blocking agent (eg. EPA 33 or diethanolamine solution). In all cases, the wash liquid is then removed.

In the preparation of the allergen coated plates, each in a respective reaction vessel, it is important to ensure that so far as possible the whole surface (both sides) of the plate reacts with the allergen. This is desirable to ensure that there is enough allergen present for the purposes of the test. It is more particularly important in the case of the rings described above (and in DE 4123324 C2) since the available surface area of the rings is less than that of the corresponding non-apertured discs.

We have found a way of ensuring that the rings are adequately coated. In particular, we have found that if the vessel is subjected to shaking, especially orbital shaking, the ring tends to rise and fall in the liquid first reagent whereby both surfaces fully contact the liquid and thus react with it. We, therefore, prefer in accordance with the invention to subject the vessel to orbital shaking during step (b) of the assay, when the plate is in the form of a ring. Indeed, the vessel is preferably subjected to orbital shaking at any time during the assay when good contact is needed between the liquid in the vessel and the surfaces of the ring. Orbital shaking will thus preferably be employed during step (c) to ensure good contact of the allergen coated plate surface with the sample under assay.

In step (c) of the assay method of the invention, the liquid sample to be assayed is added to the reaction vessel containing the reagent-coated plate. In the case of a standard allergy assay, for example, the reagent on the plate will be the respective allergen, and any IgE thereto in the sample will bind to the allergen on the plate. Subsequently, the bound antibodies to allergen will be measured directly or indirectly (step (d)). The liquid sample will normally be blood serum but other biological fluids can be used where appropriate for the analyte being assayed.

Whilst, as stated previously, the invention is of particular utility for the standard IgE assay of allergy, it is not limited to this application. For example, for certain purposes it is desirable to be able to assay for IgG₄, for example especially in the case of wasp or bee sting allergies. The invention can be used in this way. Furthermore, tests of mucosal fluid for IgA antibodies can be of use in diagnostic or therapeutic applications and the invention can be used for tests for antigen specific secretory IgA antibodies. As will be further clear to one skilled in the art, there are other applications of the invention, e.g. to the thyroid hormone assays etc., especially where a battery of similar tests is to be performed on a sample.

In step (d) of the method, the analyte (if any) bound to the plate is assayed. The procedure for this is well known in the art and will not, therefore, be described in detail herein. In the preferred assays of the invention, where the plate is a ring, a colorimetric assay is used. The optical measurement is made on a light path passing through the aperture of the ring. However, other procedures can be used, e.g. enzymic assays or radioassays, and these can be effected in conventional manner, such as by removing liquid from the vessel for analysis elsewhere, or by removing the ring for radiocounting for example.

Simply by way of example and illustration, an allergen assay of the invention could be carried out as follows. The wells of a microtitre plate are provided with activated paper rings. Then, the following steps can be performed automatically:
1) dispense 50 *µ*l of a respective liquid allergen on to the rings in each well.
2) 1 h incubation by shaking.
3) Washing (x1) with 300*µ*l washing solution which is 1% bovine serum albumin (BSA) and 0.005% Tween 20 in phosphate buffered saline (PBS).
4) dispense blocking solution 100*µ*l into each well.
5) 0.5 h incubation
6) Wash (x3) with 300*µ*l washing solution each time.
7) Dispense 50*µ* l of the standard and sample to respective wells.
8) 1 h incubation.
9) Wash (x3) with 300*µ*l washing solution.
10) Dispense 50*µ*l conjugate to each well.
11) 1 h incubation.
12) Wash (x3) with 300*µ*l washing solution.
13) Dispense 100 *µ* l enzyme substrate solution to each well.
14) 0.5 h incubation.
15) Dispense 100*µ*l stop solution to each well.
16) Take optical reading at 405 nm ref 620/630.
17) Print results in IU/ml and classes.
The total time is 4h (References to aspiration have been omitted for clarity.)

In order that the invention may be more fully understood, features thereof will now be described, by way of illustration only, with reference to the accompanying drawings, wherein:
Fig. 1 is a schematic illustration of an embodiment of the method of the invention;
Fig. 2 is a vertical sectional view of a solid support in a reaction vessel; and
Fig. 3 is a standard curve for the assay described hereinafter in Example 3.

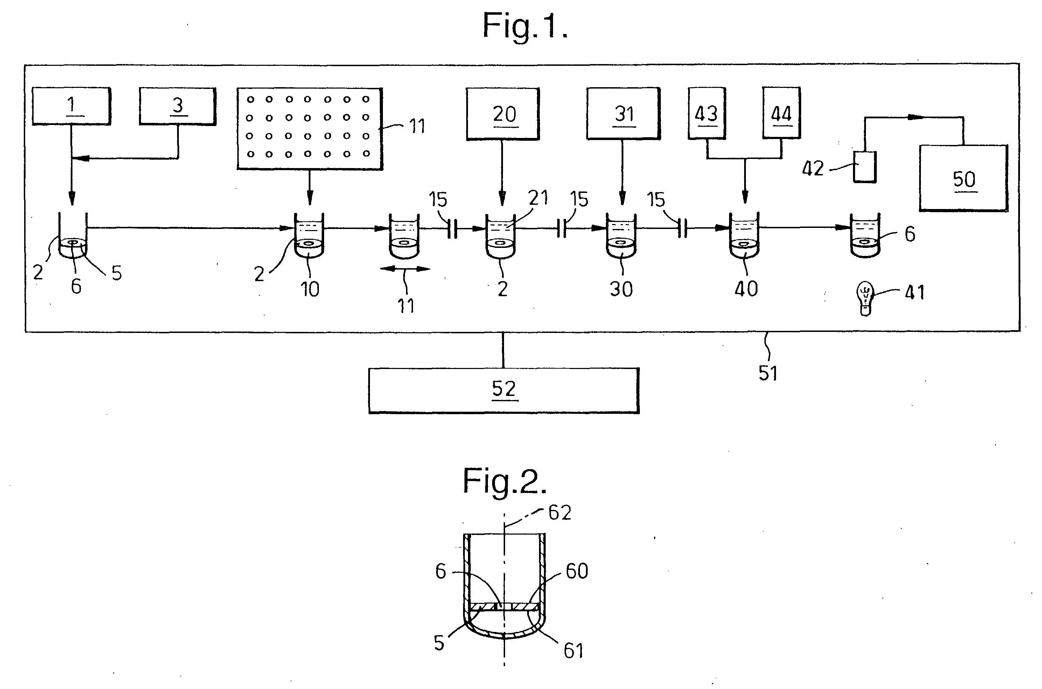

Referring to Fig. 1, there is shown schematically one system for carrying out the method of the invention. The system comprises a source or store 1 of reaction vessels 2, and a store 3 of plates, eg. discs or rings. As illustrated, the plate is a paper disc 5 with central aperture 6. Means (not shown) are provided to deliver one disc 5 into each reaction vessel 2.

The vessel 2 then receives an appropriate quantity of first reagent 10 selected from a store 11 of all the first reagents which may be required for a particular assay being conducted. A conventional remote control aspirator and delivery probe (not shown) can be used to withdraw the selected first reagent 10 from its store and deliver it into the vessel 2.

Vessel 2 is then subjected to orbital shaking in a shaker 11, and this is continued for an appropriate time (for example 30 to 60 minutes) for the first reagent 10 to become bound to the disc 5. If further reagents are necessary in order for the first reagent 10 to bind to the disc 5, these can be automatically provided to vessel 2 before or after the first reagent, as desired.

After the first reagent 10 has become bound to both faces of the disc 5, the liquid is aspirated from the vessel 2 and the disc washed. This operation is indicated by symbol 15 in Fig. 1.

Next, a blocking solution 21 is dispensed from reservoir 20 into vessel 2 to block the remaining active sites on disc 5. The tube is then aspirated and washed 15.

An aliquot of sample 30 is then dispensed into vessel 2 from reservoir 31, and reaction between the analyte of interest therein (if any) and the first reagent 10 takes place. The vessel may be shaken and/or incubated as appropriate.

After reaction, liquid in the vessel 2 is removed by aspiration and the disc 5 is washed 15. Thereafter the presence and/or amount of analyte bound to disc 5 is assayed conventionally. Thus, one or more liquid assay reagents 40 are dispensed into vessel 2 (from reservoirs 43,44), and when the reaction is complete, a colorimetric determination is effected using a light source 41 and sensor 42. The light path is generally axial of the vessel 2, through the aperture 6 in disc 5. The results may be printed or displayed in a unit 50.

The system 51 overall is controlled by a computer 52, so that the whole procedure can be automated.

Fig. 2 illustrates a reaction vessel with a paper disc 5 therein. The disc has a central aperture 6 therein. Whilst the disc 5 is of a diameter only slightly less than that of the vessel, and is thus a close fit therein, nevertheless both the top 60 and bottom 61 surfaces will take part in the assay by binding to the first reagent. Thus, when the vessel is subjected to orbital shaking, the disc tends to rise from the bottom of the vessel and rotate, the liquid being turbulent around the aperture 6. As a result, both surfaces of the disc fully contact the liquid for reaction therewith or for washing, as the case may be. Because the disc 5 is a close fit in the vessel, the aperture 6 is automatically centered in the vessel, so ensuring a light path 62 generally axially of the vessel.

In order that the invention may be more fully understood, the following Examples are given by way of illustration only.

### Example 1

### Activation of paper rings

Paper rings (6.3mm diameter) having an accurately centred aperture (2.0mm ± 0.1mm diameter) were soaked in potassium phosphate (K₃PO₄) buffer (pH 11.9) for 10 minutes in a sintered glass funnel at room temperature. The rings were then washed with more potassium phosphate buffer. The washed rings were then placed in a glass beaker with 100ml of the potassium phosphate buffer per 1000 rings. The mixture was stirred to move the rings in the buffer until the pH stabilised. Over a period of about 1 minute, a solution of 0.435g cyanogen bromide in 10.0ml acetonitrile was slowly added, per 1000 rings, the stirring being continued. The pH of the mixture was maintained between 11.5 and 12.0 over a period of 10 minutes after addition of the cyanogen bromide, by the addition of caustic soda.

The activated rings were then transferred to a sintered glass funnel on an Erlenmayer flask and washed with water. The washed rings were then blotted dry with filter paper and placed in a plastics bag connected to a dry nitrogen gas line. Gas was passed through the bag to dry the activated rings. Once the rings had returned to substantially their original dry weight, they were sealed in a plastics bag and stored in an evacuated desiccator at about -20°C.

### Example 2

### Binding first reagent to activated rings

Individual activated rings as made in Example 1 were placed in microtitre plate wells (one ring per well) and 50*µ*l of a solution of an allergen (containing a peroxide accelerator) were dispensed into each well. The wells were subjected to orbital shaking for a period of about 1 hour, after which the liquid in each well was removed by aspiration. The ring in each well was then washed with 300 ml washing solution (1% BSA, 0.005% Tween 20 in PBS). After removal of the washing solution by aspiration, 100*µ* l of blocking solution was dispensed into each well to react with any reactive sites remaining on the surfaces of the ring. After incubating for 30 minutes, the solution was removed by aspiration and the rings were washed three times with 300*µ*l aliquots of the washing solution. The rings were then ready for use in assay.

The above procedure is applicable to all the common allergens. The allergen solutions used are standard for each allergen (as will be known to those in the art). The usual accelerator is peroxyacetic acid.. The blocking solution was diethanolamine (pH 9.8).

### Example 3

### Assay

Assays for various allergens present in a sample were carried out as follows.

Microtitre plates were used containing, in each well, an activated paper ring prepared according to Example 1. Then, into each well, was dispensed 50*µ*l of a solution of an allergen to bind the allergen to the paper ring, as described in

### Example 2.

In order to carry out the assay and to derive the required standard curve, 50*µ*l aliquots of IgE standards (human IgE in PBS with 10% horse serum) and of the samples to be analysed, were dispensed into respective individual wells containing the appropriate first reagent paper ring. (For total IgE measurements, rabbit anti (human IgE) was used (Dako).) The wells were incubated for 1 hour, with orbital shaking, and thereafter the liquid was aspirated therefrom and the paper ring washed three times with 300*µ*l aliquots of washing solution.

At this stage in the assay, in each well the relevant IgE has been bound to the respective ring. In the next stage of the assay, the amount of IgE was quantified. There was added to each well 50*µ*l of a goat anti-human IgE conjugated with a marker enzyme in PBS with 1% BSA, Mg, Zn and some stabiliser. The enzyme used in this Example was alkaline phosphatase. After 1 hour incubation, the rings were washed three times with 300*µ*l aliquots of washing solution. There was then dispensed into each well a 100*µ*l aliquot of a substrate solution, being PNPP for the enzyme alkaline phosphatase. After 30 minutes incubation, 100*µ*l of a stop solution (in this case 2N caustic soda) were dispensed into each well to stop the enzyme reaction.

The colour (optical density) of the liquid in each well was then measured at 405nm. From the wells in which standards had been assayed, a calibration or standard curve was plotted. From this curve, the quantities of the IgE-antibodies to the various allergens tested in the sample could be determined from the OD results from the respective sample wells.

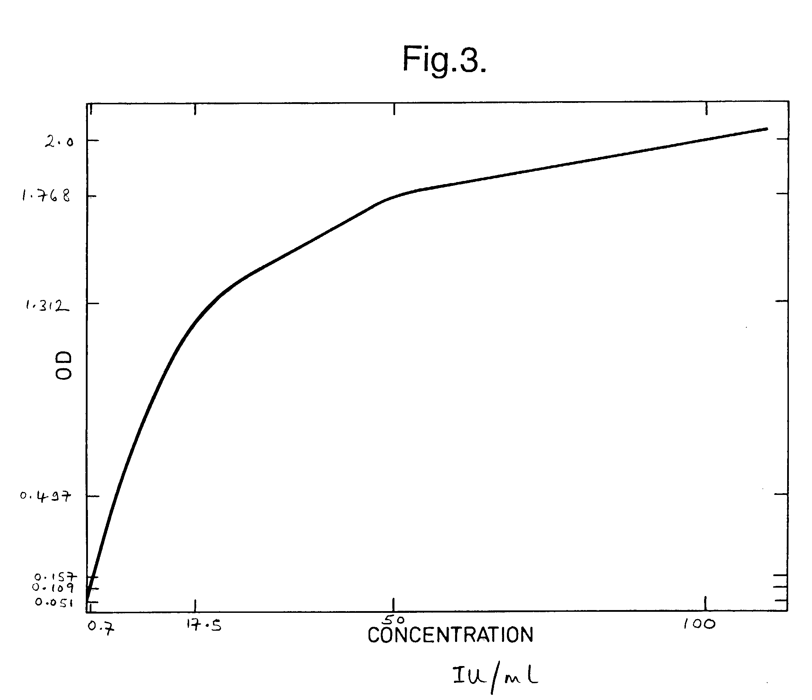

Fig. 3 is a standard curve plotted from the results shown. The full assay results including those for the standards used for the construction of the standard curve and also results on a patient sample for various allergens are set out in the following Table. The sample was tested for allergens D1, allergen m1, allergen e1, and allergen t3. In the conventional way, serial dilutions are conducted as indicated. In the Table:
d1 = Dermatophagoides pteronyssinus
m1 = Altemaria tenuis
e1 = Cat hair + epithelium
t3 = Birch pollen
m2 = Aspergillus amstelodami
m50 = Aspergillus flavus
m15 = Cladosporium herbarum
m16 = Curvularia lunata

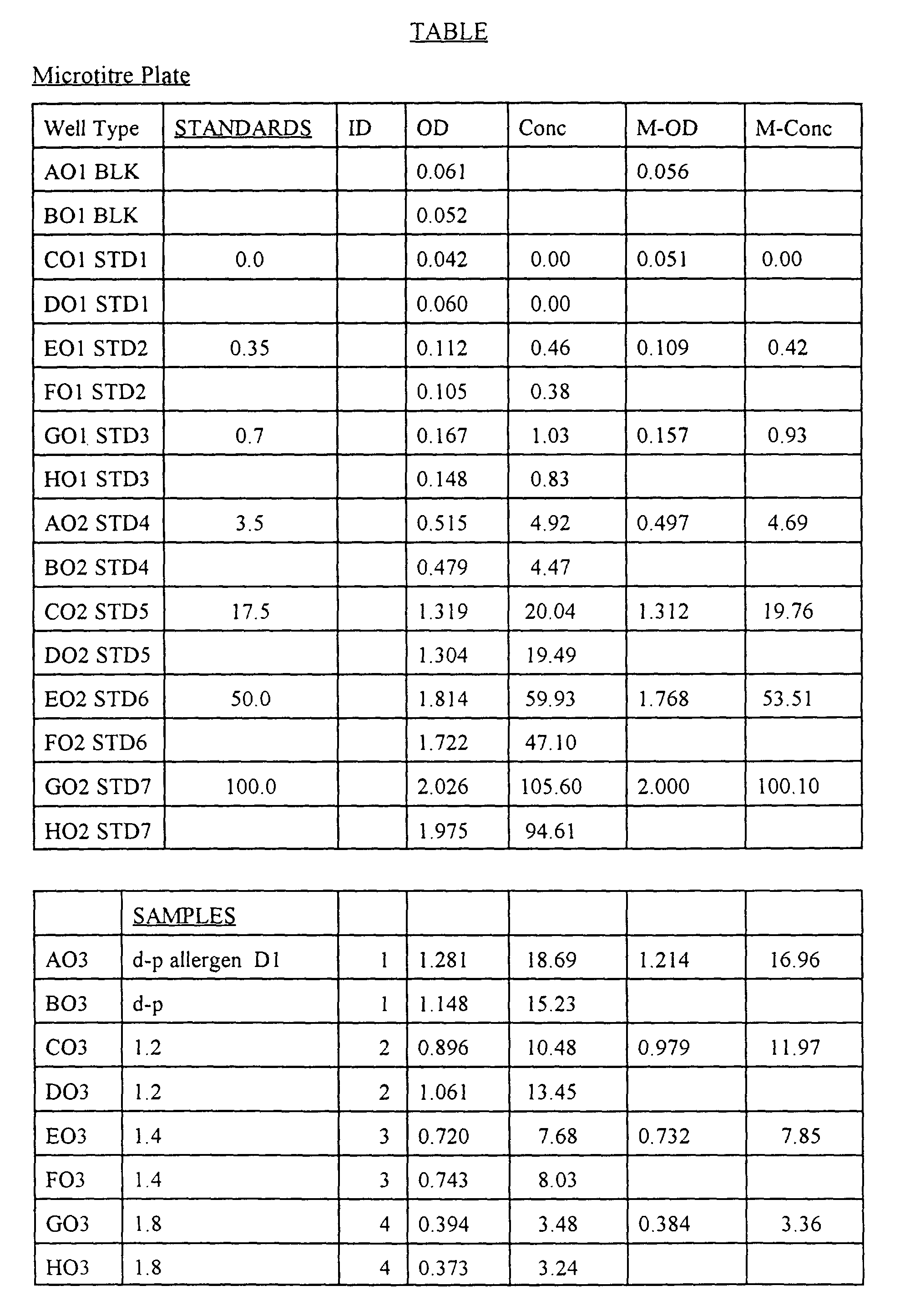

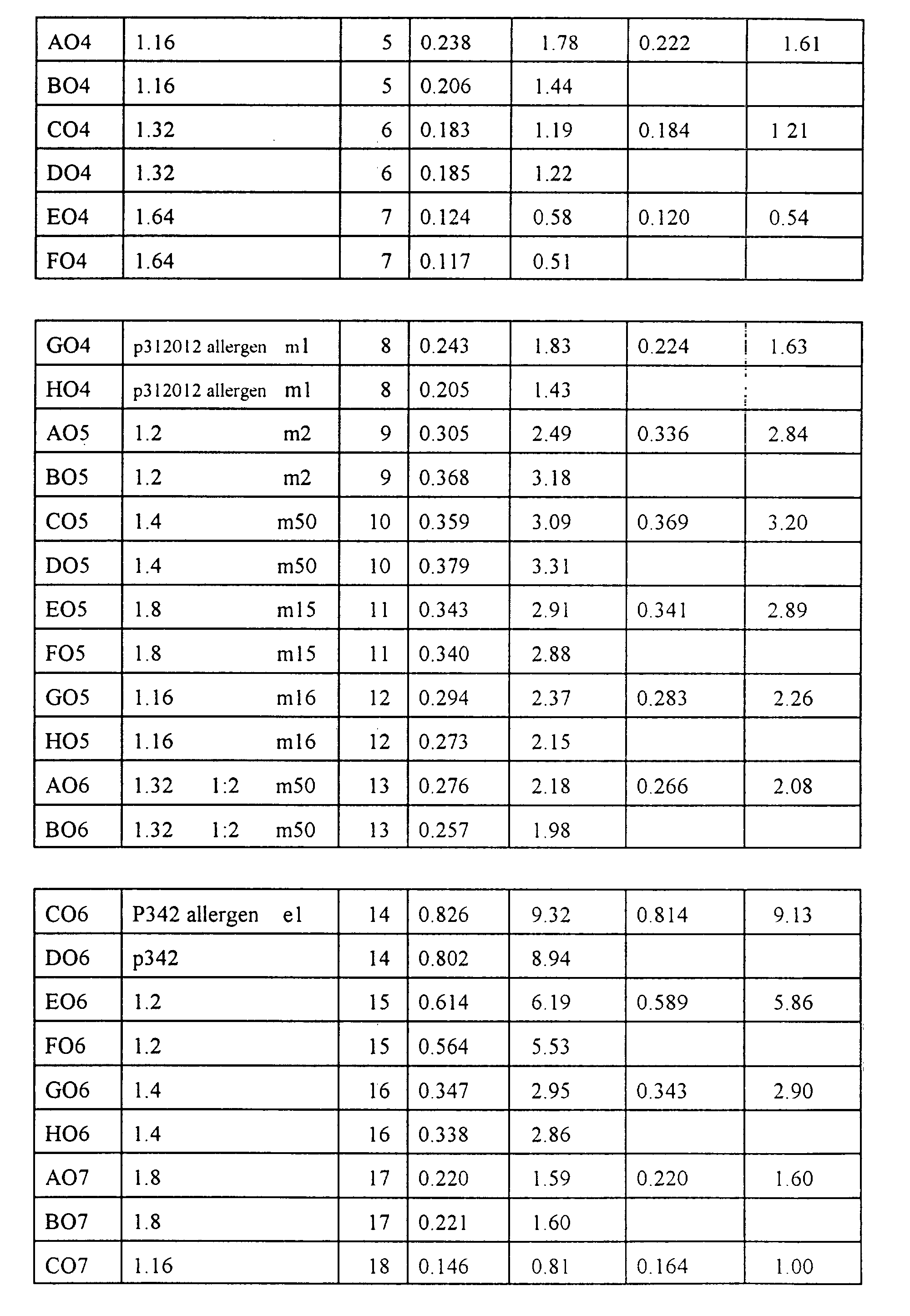

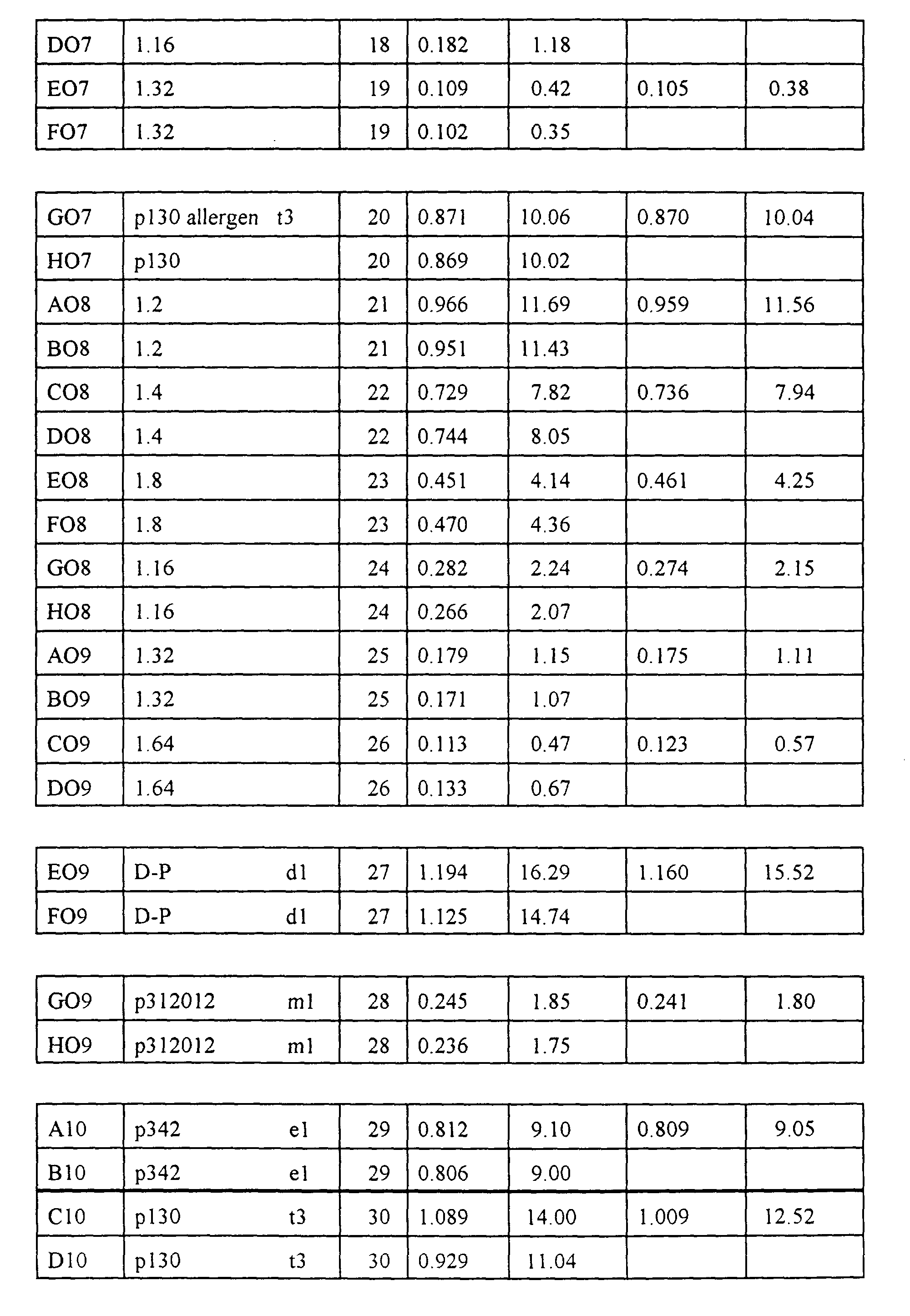

### Example 4

### Assay of IgE to D. pteronyssinus

Paper rings activated as described in Example 1 were placed in a microtitre well (one ring to each well) and 50*µ*l of Dermatophagoides pteronyssinus extract containing 0.05% (w/v) peroxyacetic acid was added to each well. After incubation for 1h with orbital shaking at room temperature, the rings were each washed once with 300*µ*l of the washing solution described in Example 2. Then, 100*µ*l of the blocking/neutralising solution (diethanolamine) described in Example 2 was dispensed into each well. After incubation for 30 minuteswith orbital shaking at room temperature, the rings were each washed 3 times with 300*µ*l washing solution each wash.

The rings were then used in the assay as follows. Each well had 50*µ*l of a standard or a sample dispensed therein. After incubation for 1 h with orbital shaking at room temperature, the rings were washed 3 times with 300*µ*l washing solution each wash. Then into each well is dispensed 50*µ* l conjugate as described in Example 3. After incubation for 1 h with orbital shaking at room temperature, the rings were washed 3 times with 300*µ*l washing solution each wash. Then, into each well was dispensed 100*µ*l substrate solution described in Example 3. After incubation for 30 minutes at room temperature, 100*µ*l stop solution was dispensed into each well. Then, the optical density of liquid in each well was read at 405 nm ref 620/630. A curve of optical density versus the standard concentration was plotted and used to read the concentration of specific IgE in each sample.

## Claims

1. A method of immunoassay of an analyte in a liquid sample, which comprises the steps of:
a) providing a plate in a vessel which plate will react when brought into contact with liquid first reagent in step (b);
b) adding to the vessel liquid first reagent which reacts with the surface of the plate to become bound thereto;
c) adding the liquid sample containing the analyte under assay to the vessel, whereby the analyte is immunospecifically bound directly or indirectly with said first reagent on said plate; and
d) directly or indirectly assaying the analyte bound to the plate.

2. A method according to claim 1, wherein the plate is a disc of paper or plastics material with an activated surface.

3. A method according to claim 2, wherein the disc has a central aperture.

4. A method according to claim 3, wherein in steps (b) and (c), the vessel is subjected to orbital shaking.

5. A method according to claim 1, 2, 3 or 4, wherein step (d) is conducted on contents in the vessel.

6. A method according to claim 1, 2, 3, 4 or 5, wherein the immunoassay is for the diagnosis of allergy, and wherein the first reagent is an allergen, and the analyte if an IgE immunospecific thereto.

7. A method according to any of claims 1 to 6, wherein, in step (a), a plate that has been activated to make it autoreactive with said first reagent, is placed in said vessel.

8. A method according to any of claims 1 to 7, wherein a single liquid sample is assayed for a plurality of individual analytes therein, and wherein steps (a) to (d) are effected in respect of each analyte in a respective vessel.

9. A method according to any of claims 1 to 8, which is effected automatically under computer control.

10. A method according to claim 6, wherein the liquid sample is blood serum.

11. A method according to claim 3, wherein the analyte bound to the disc in the vessel is directly or indirectly assayed by optical analysis through the aperture in the disc.

12. A method according to claim 2, wherein step (d) is conducted without removing the disc from the vessel.

13. An immunoassay system for continuously conducting a plurality of identical or similar tests on a biological fluid, for a plurality of analytes therein, wherein in each test, sample is contacted with a selected specific reagent for that test bound to an insoluble plate, which system comprises a plurality of reaction vessels each containing an activated plate; a plurality of a liquid first reagents; means for dispensing a selected said first reagent into each said vessel; means for agitating the vessels; means for dispensing liquid sample into each vessel; and means for assaying a component of the mixture in the vessel; and wherein said dispensing means and assaying means are controlled automatically by computer.

## Patentansprüche

1. Methode zur Durchführung eines Immunoassays an einem Analyten in einer Flüssigprobe, welche Methode folgende Schritte umfasst:
a) Bereitstellen einer Platte in einem Gefäß, welche Platte reagiert, wenn sie mit dem ersten Flüssigreagenzes in Schritt (b) in Berührung gebracht wird;
b) Eingeben in das Gefäß eines ersten Flüssigreagenzes, das mit der Oberfläche der Platte reagiert und dabei an diese bindet;
c) Eingeben der den zu untersuchenden Analyten enthaltenden Flüssigprobe in das Gefäß, wobei der Analyt immunospezifisch direkt oder indirekt mit dem ersten Reagenz auf der Platte bindet; und
d) direkte oder indirekte Untersuchung des an der Platte gebundenen Analyten.

2. Methode nach Anspruch 1, bei der die Platte eine Scheibe aus Papier oder Kunststoffmaterial mit einer aktivierten Oberfläche ist.

3. Methode nach Anspruch 2, bei der die Scheibe eine zentrale Öffnung aufweist.

4. Methode nach Anspruch 3, bei der das Gefäß in den Schritten (b) und (c) einem orbitalen Schütteln unterworfen wird.

5. Methode nach Anspruch 1, 2, 3 oder 4, bei der der Schritt (d) am Gefäßinhalt durchgeführt wird.

6. Methode nach Anspruch 1, 2, 3, 4 oder 5, bei der der Immunoassay der Diagnose einer Allergie dient, und bei der das erste Reagenz ein Allergen ist und der Analyt ein hierfür immunospezifisches IgE ist.

7. Methode nach einem der Ansprüche 1 bis 6, bei der in Schritt (a) eine Platte, die aktiviert worden ist, um sie mit dem ersten Reagenz autoreaktiv zu machen, in das Gefäß gegeben wird.

8. Methode nach einem der Ansprüche 1 bis 7, bei der eine einzige Flüssigprobe auf eine Anzahl verschiedener darin enthaltener Analyte untersucht wird und bei der die Schritte (a) bis (d) bezüglich jeden Analyten in einem entsprechenden Gefäß durchgeführt werden.

9. Methode nach einem der Ansprüche 1 bis 8, die automatisch unter Rechnersteuerung durchgeführt wird.

10. Methode nach Anspruch 6, bei der die Flüssigprobe Blutserum ist.

11. Methode nach Anspruch 3, bei der der an die Scheibe im Gefäß gebundene Analyt durch optische Analyse durch die Öffnung in der Scheibe direkt oder indirekt untersucht wird.

12. Methode nach Anspruch 2, bei der Schritt (d) ohne Entfernen der Scheibe aus dem Gefäß ausgeführt wird.

13. Immunoassay-System für die kontinuierliche Durchführung einer Anzahl verschiedener identischer oder ähnlicher Tests an einem biologischen Fluid auf eine Anzahl verschiedener darin enthaltener Analyte hin, bei dem bei jedem Test eine Probe mit einem ausgewählten für diesen Test spezifischen Reagenz in Kontakt gebracht, das an eine unlösliche Platte gebunden ist, welches System eine Anzahl verschiedener Reaktionsgefäße umfasst, von denen jedes eine aktivierte Platte enthält; eine Anzahl verschiedener erster Flüssigreagenzien; eine Vorrichtung für das Eingeben eines ausgewählten ersten Reagenzes in jedes Gefäß; eine Vorrichtung für das Rühren der Gefäße; eine Vorrichtung für das Eingeben der Flüssigprobe in jedes Gefäß; und eine Vorrichtung für das Untersuchen einer Komponente der Mischung im Gefäß; und bei dem die Eingabevorrichtung und die Untersuchungsvorrichtung automatisch durch einen Rechner gesteuert werden.

## Revendications

1. Un procédé d'immuno-essai d'un analyte dans un échantillon liquide, qui comporte les étapes suivantes :
a) prévision d'une plaque, dans un récipient, qui va réagir lorsque mise en contact avec un premier réactif liquide pendant l'étape (b) ;
b) ajout, dans le récipient, du premier réactif liquide qui réagit avec la surface de la plaque pour devenir lié à celle-ci ;
c) ajout d'un échantillon liquide contenant l'analyte à l'essai dans le récipient, de sorte que l'analyte est immunospécifiquement lié, directement ou indirectement, avec ledit premier réactif sur ladite plaque ; et
d) analyse directe ou indirecte de l'analyte lié à la plaque.

2. Un procédé selon la revendication 1, selon lequel la plaque est un disque de papier ou de matière plastique avec une surface activée.

3. Un procédé selon la revendication 2, selon lequel le disque a une ouverture centrale.

4. Un procédé selon la revendication 3, selon lequel, pendant les étapes (b) et (c), le récipient est soumis à des secousses orbitales.

5. Un procédé selon la revendication 1, 2, 3 ou 4, selon lequel l'étape (d) est menée sur le contenu du récipient.

6. Un procédé selon la revendication 1, 2, 3, 4 ou 5, selon lequel l'immuno-essai porte sur le diagnostic d'une allergie, et selon lequel le premier réactif est un allergène, et l'analyte est une IgE immunospécifique de celui-ci.

7. Un procédé selon l'une quelconque des revendications 1 à 6, selon lequel, pendant l'étape (a), une plaque qui a été activée pour la rendre autoréactive avec ledit premier réactif, est placée dans ledit récipient.

8. Un procédé selon l'une quelconque des revendications 1 à 7, selon lequel un échantillon liquide unique est analysé pour détecter une pluralité d'analytes individuels à l'intérieur de l'échantillon, et selon lequel les étapes (a) à (d) sont menés relativement à chaque analyte dans un récipient respectif.

9. Un procédé selon l'une quelconque des revendications 1 à 8, qui est mené automatiquement sous commande informatisée.

10. Un procédé selon la revendication 6, selon lequel l'échantillon liquide est un sérum sanguin.

11. Un procédé selon la revendication 3, selon lequel l'analyte lié au disque dans le récipient est analysé directement ou indirectement par analyse optique à travers l'ouverture dans le disque.

12. Un procédé selon la revendication 2, selon lequel l'étape (d) est menée sans extraire le disque hors du récipient.

13. Un système d'immuno-essai pour mener continuellement une pluralité de tests identiques ou similaires sur un liquide biologique, pour détecter une pluralité d'analytes à l'intérieur de celui-ci, dans lequel, pendant chaque test, un échantillon est mis en contact avec un réactif spécifique sélectionné pour ce test, lié à une plaque insoluble, ledit système comportant une pluralité de récipients de réaction contenant chacun une plaque activée ; une pluralité de premiers réactifs liquides ; des moyens de distribution d'un dit premier réactif liquide sélectionné dans chacun desdits récipients ; des moyens d'agitation des récipients ; des moyens de distribution d'un échantillon liquide dans chaque récipient ; et des moyens d'analyse d'un composant du mélange à l'intérieur du récipient ; et dans lequel lesdits moyens de distribution et les moyens d'analyse sont contrôlés automatiquement par un ordinateur.
